# EUROPEAN PATENT APPLICATION

(11) **EP 3 650 530 A1**
(43) Date of publication of application: **13.05.2020**
(21) Application number: 18827422.9
(22) Date of filing: 02.07.2018
(51) Int. Cl.: C12M 1/00, C12M 1/14, C12M 3/00

(54) **CULTURING DEVICE, CARRIER, AND CULTURE SUBJECT COLLECTION METHOD**

(30) Priority: 04.07.2017 JP 2017131025; 28.05.2018 JP 2018101710
(71) Applicant: Nippon Soda Co., Ltd., Tokyo 100-8165 (JP)
(72) Inventor: TSUZUKI Mikio, Hachiouji-shi Tokyo 192-0392 (JP); IWAKOSHI Banri, Tokyo 100-8165 (JP)
(74) Representative: Wibbelmann, Jobst
(86) International application number: PCT/JP2018/024988
(87) International publication number: WO 2019/009221

(57) **Abstract**

A culturing device (1) includes a carrier unit having a carrier (2) to which a culture subject adheres, a supply unit (3) that supplies a culture solution to the carrier (2), and a reservoir unit (5) in which the culture solution flowing out from the carrier (2) is stored. The carrier (2) includes a base (21) and a plurality of protrusions (23) which are formed on the surface of the base (21) and of which a relative position of each tip (233) with respect to the base (21) can be changed. The carrier unit includes a swinging mechanism (27) that swing the the carrier (2) and changes the relative positions of the protrusions (23).

## Description

### Technical Field

The present invention relates to a culturing device for culturing a culture subject such as microalgae, a carrier, and a culture subject collection method.

Priority is claimed on Japanese Patent Application No. 2017-131025, filed July 4, 2017, and Japanese Patent Application No. 2018-101710, filed May 28, 2018, the contents of which are incorporated herein by reference.

### Background Art

Regarding measures against global warming, efforts for reducing emission of global warming gases as much as possible are strongly demanded for industries in all countries. Microalgae such as *Chlorellae* and microorganisms such as photosynthetic bacteria are considered to be very promising resources that can produce energy without emission of CO₂ and other industrially available resources, and they are expected to be utilized on a commercial level and efficiently produced.

In order to utilize microalgae such as *Chlorellae* for energy resources and other industrial uses, it is necessary to produce them at as low a cost as possible. However, when a large amount of microalgae are cultured in water, large pools and tanks are necessary. Thus, there are problems of costs increasing due to securing of sites or large-scale facilities.

Patent Literature 1 proposes a culture system in which, in order to effectively utilize land and increase a production amount per unit area with simple facilities, a culture solution is caused to naturally flow down on the surface of a vertically erected carrier, microorganisms such as microalgae continuously grow on the surface of the carrier, and the microorganisms are continuously collected from the culture solution that has naturally flowed down. In this system, a thin water film on the surface of the carrier corresponds to a pool water surface in the conventional method, light (artificial light), carbon dioxide gas, and nutrients are obtained, and photosynthesis is performed. In the unit including this system, a culture amount equal to or larger than that of the same area of the water surface is obtained with a single carrier, and according to a parallel multi-layer facility for the carrier, a harvesting amount of 10 to 20 times that of the same floor area in a conventional method such as a pool can be expected. In addition, when the units are vertically laminated, securing of a culture amount of 100 times that of the same floor area in a conventional procedure can be expected. According to such a culture system, it is possible to overcome restrictions of location that are limited to areas with abundant sunlight, and culturing thus becomes possible in polar areas, underground, and also in outer space.

### [Citation List]

### [Patent Literature]

### [Patent Literature 1]

Japanese Unexamined Patent Application, First Publication No. 2013-153744

### SUMMARY OF INVENTION

### Technical Problem

However, the culture system described in Patent Literature 1 has problems in that a microorganism culture rate is low, about one month is required from when the culture starts until microorganisms can be regularly produced, and thus the production efficiency is poor.

An object of the present invention is to provide a culturing device, a carrier, and a culture subject collection method through which it is possible to efficiently produce microorganisms on the surface of and inside a carrier.

### Solution to Problem

A culturing device according to a first aspect of the present invention includes a carrier unit having a carrier to which a culture subject adheres, a supply unit that supplies a culture solution to the carrier, and a reservoir unit in which the culture solution flowing out from the carrier is stored. The carrier includes a base and a plurality of protrusions which are formed on the surface of the base and of which a relative position of each tip (apex, point) with respect to the base is able to be changed. The carrier unit includes a swinging mechanism that swings the carrier and changes the relative positions of the protrusions as the carrier is swung.

The base may be provided in (along) a vertical direction and the tips of the protrusions may be positioned below roots of the protrusions.

The culture solution supplied from the supply unit to the carrier may flow from the roots of the protrusions toward the tips.

The protrusions may be fibrous members that extend from the base.

The fibrous members may have annular parts (or loop, ring, circle).

The swinging mechanism may include a storage unit in which the carrier is stored, and an ultrasonic vibration unit that applies an ultrasonic vibration to the carrier stored in the storage unit.

The swinging mechanism may include a contact member that is provided in contact with the surface of the carrier, and a moving unit that moves the contact member along the surface.

A culturing device according to another aspect of the present invention includes a carrier to which a culture subject adheres, a supply unit that supplies a culture solution to the carrier, and a reservoir unit in which the culture solution flowing out from the carrier is stored, and a water capacity per unit area of the carrier is 0.2 g/cm² or more.

According to still another aspect of the present invention, a carrier used for a culturing device is provided, including a carrier to which a culture subject adheres, a supply unit that supplies a culture solution to the carrier, and a reservoir unit in which the culture solution flowing out from the carrier is stored. The carrier includes a base having a sheet form; and a plurality of protrusions which are formed on the surface of the base and of which a relative position of each tip with respect to the base is able to be changed, and the tips of the protrusions are able to be positioned below roots of the protrusions.

A culture subject collection method according to yet another aspect of the present invention includes a step of adhering a culture subject to a carrier including a base and a plurality of protrusions which are formed on the surface of the base and of which a relative position of each tip with respect to the base is able to be changed, a step of culturing the culture subject in the carrier, a step of changing the relative positions of the protrusions by swinging the carrier and separating the culture subject from the carrier, and a step of collecting a fluid containing the culture subject separated from the carrier.

### Advantageous Effects of Invention

According to the above aspects, microorganisms can grow on the surface of and inside the carrier and microorganisms can be efficiently produced and collected.

### BRIEF DESCRIPTION OF DRAWINGS

Fig. 1 is a perspective view schematically showing a microorganism culture system according to a first embodiment of the present invention.
Fig. 2 is a side view schematically showing the microorganism culture system according to the first embodiment of the present invention.
Fig. 3A is an enlarged cross-sectional view of a carrier according to a second embodiment.
Fig. 3B is an illustrative diagram of a protrusion part of the carrier.
Fig. 4 is a schematic configuration diagram of a hanger according to the second embodiment.
Fig. 5 is a flowchart illustrating a microorganism culture collection method according to the second embodiment.
Fig. 6 is a schematic configuration diagram of a net vibration mechanism according to a third embodiment.
Fig. 7A is an enlarged view of a net component and a carrier.
Fig. 7B is a cross-sectional view along the line VIIb-VIIb in Fig. 7A.
Fig. 8 is a schematic configuration diagram of an ultrasonic vibration mechanism according to a fourth embodiment.
Fig. 9 is a perspective view of a vibration tank according to the fourth embodiment.

### DESCRIPTION OF EMBODIMENTS

Hereinafter, a microorganism culture system according to embodiments of the present invention will be described with reference to the drawings.

### [First embodiment]

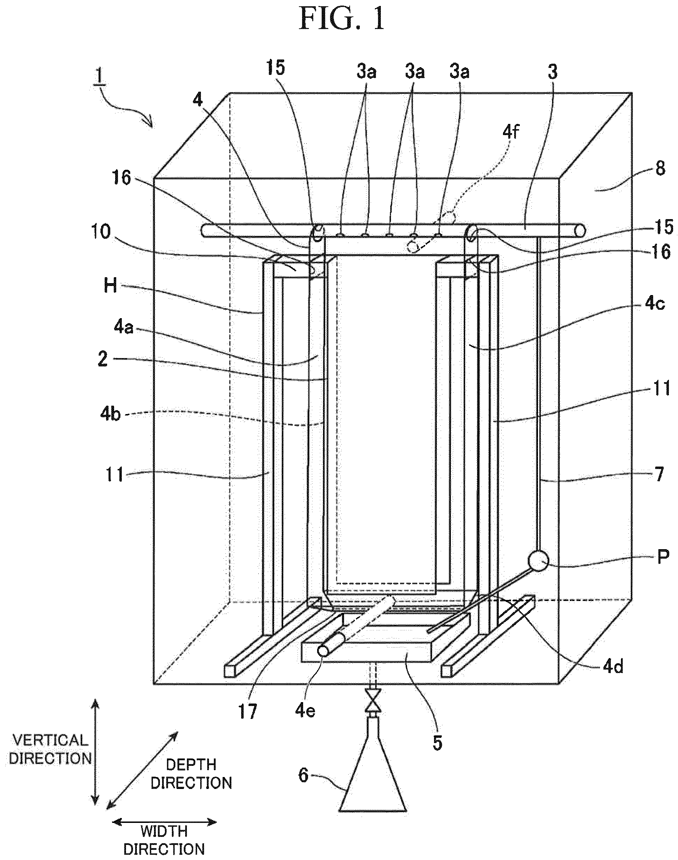

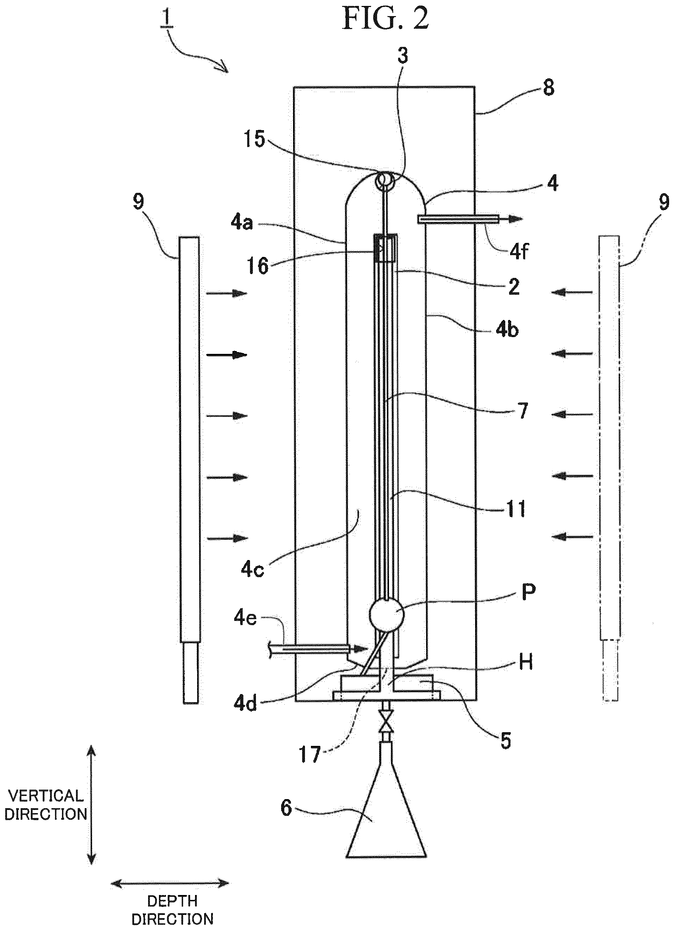

As schematically shown in Fig. 1 or Fig. 2, a culture system 1 according to the present embodiment, which is a system for culturing microorganisms in a gas phase, includes a sheet-like carrier 2 disposed in a vertical direction, a culture solution supply unit 3 configured to supply a culture solution to the carrier 2, a sheet component (inner cover member) 4 which covers a surface of the carrier 2, an effluent tank 5 in which a culture solution containing microorganisms flowing out from the carrier 2 is stored, a harvest container 6 in which microorganisms separated from the culture solution stored in the effluent tank 5 are stored, a circulation flow path 7 through which the culture solution separated from the culture solution stored in the effluent tank 5 circulates, a case (outer cover member) 8 covering the carrier 2, the culture solution supply unit 3, the sheet component 4, the effluent tank 5, the circulation flow path 7, and a light emission unit 9 configured to emit light to the carrier 2.

In the following description, the vertical direction of the culture system 1 will be simply referred to as a vertical direction. The width direction of the carrier 2 provided in the culture system 1 will be simply referred to as a width direction. A direction intersecting the vertical direction and the width direction in the culture system 1 will be simply referred to as a depth direction.

The carrier 2 is a belt-like sheet having a certain width, and to which microorganisms can adhere and which allows a culture solution supplied from above to penetrate thereinto and flow down, and the water capacity per unit area is preferably 0.2 g/cm² or more. The term "water capacity" in this specification means a value (g/cm²) measured in a water retention test that will be described below in an example. The water capacity per unit area of the carrier 2 is preferably 0.25 g/cm² or more and more preferably 0.3 g/cm² or more. The upper limit of the water capacity per unit area of the carrier 2 is not particularly limited but it can be selected from among 10 g/cm² or less, 8 g/cm² or less, 5 g/cm² or less, 3 g/cm² or less, and 1 g/cm² or less.

Specific examples of the carrier 2 include pile fabrics of twisted yarns or untwisted yarns, any of which can be used, and an untwisted yarn pile fabric is particularly preferable. The material of the pile fabric is not particularly limited, and specific examples thereof include natural fibers (plant fibers or animal fibers) such as cotton, silk, fur, wool, and hemp, and synthetic fibers such as acrylic, polyester, nylon, vinylon, polyolefin, and polyurethane. Piling is a type of weaving and refers to weaving in which loop-like fibers (loop of thread) protrude from a fabric ground weave in vertical and horizontal directions at certain intervals, and cover the surface of the ground weave, and the loop of thread has elasticity. The pile fabric is a pile-woven fabric. The lengths of the piles of the pile fabric are not particularly limited, and can be 3 mm to 30 mm. When a pile fabric is used as the carrier 2, appropriate water retention properties and drain properties are obtained, microorganisms can remain and be grown on the loop of thread appropriately, some of the grown microorganisms can be appropriately shaken off from the loop of thread and discharged, and a culture system with high efficiency can be constructed.

The carrier 2 of this embodiment is consisted of a sheet having a rectangular sheet form. However, the shape of the carrier 2 is not limited to a flat plate shape, and may be a cylindrical shape or a rectangular tube shape in which both ends in the longitudinal direction or the width direction are connected. In addition, the number of carriers 2 is not limited to one, and two or more carriers may be provided.

The carrier 2 is hung on a horizontal part of the upper end of a hanger H in an inverted U shape, that is, while being folded in half from the center, and is installed by being suspended with the end of the carrier 2 that is fixed to the hanger H with a fastener such as a clip and a hook.

The hanger H is a member that allows the carrier 2 to be suspended at a desired height (for example, about 1 m) from a lower end of the hanger H, and includes a rod-like fixing member 10 that is disposed in a horizontal direction on which to hang or fasten the carrier 2 and a pair of leg members 11 that support both ends of the fixing member 10. The hanger H may have a plurality of fixing members 10 that are parallel to each other.

In addition to the above method, the carrier 2 may be installed by a method such as attaching it to a support member such as a rigid frame in a self-supporting manner, or providing a fastener or the like directly below the culture solution supply unit 3 to be described below and suspending it on the fastener.

When a fastener or the like is provided directly below the culture solution supply unit 3 to be described below and the carrier 2 is suspended on the fastener, the fastener can be used as a flow path through which a culture solution is supplied to the carrier 2 and the carrier 2 can be reliably installed directly below the culture solution supply unit 3, and thus it is not necessary to align positions of the culture solution supply unit 3 and the hanger H.

The culture solution supply unit 3 of this embodiment is a horizontally disposed tubular member through which a culture solution is discharged and is supplied to the carrier 2, and a part thereof is connected to a culture solution reservoir tank and nutrient supply tank which are not shown via the circulation flow path 7. In the culture solution supply unit 3, on the peripheral wall of the center that faces the upper end of the carrier 2, a plurality of supply holes 3a from which a culture solution is discharged are formed at certain intervals in the axial direction. The supply holes 3a are disposed downward, and allow a culture solution to be supplied to the upper end of the carrier 2 so that water content is almost uniform over the entire carrier 2 in the width direction. A plurality of culture solution supply units 3 may be disposed according to the number or dispositions of carriers 2.

A culture solution supply capacity of the culture solution supply unit 3 is preferably a capacity at which a downflow velocity of the culture solution in the carrier 2 can be 5 mL/h/m² or more and 30,000 mL/h/m² or less. This variable range corresponds to the growth of microorganisms. For example, one *Chlorella* cell grows and divides into 4 cells and each grows to the size before the division in 16 hours. At the beginning of division, a small amount of nutrients is enough, but it is necessary to provide a sufficient amount thereof during a growth period. Therefore, microorganisms can be made to continuously naturally flow down together with a culture solution while maintaining the growth by always filling the surroundings of microorganisms with a fresh culture solution. In addition, when the flow rate of the culture solution is changed or an impact such as vibration is applied to the carrier 2 at a certain time, and thus a surface part of the microorganism layer adhered to the carrier 2 is forcibly dropped, photosynthesis in the lower layer part becomes active, microorganisms grow, and the yield can be increased.

In order to provide the minimum water and/or nutrients necessary for maintaining stable cell proliferation of microorganisms and facilitating gas (CO₂) exchange, although it depends on the amount of microorganisms seeded, for example, if the carrier 2 is made of a sheet component that is not a pile fabric of 0.5 m² or more, it is necessary to cause a culture solution to flow at an initial flow rate of 1,000 mL/h/m² or more and then to gradually increase the flow rate to 5,000 mL/h/m². Therefore, an amount of microorganisms flowing out also increases as the flow rate increases until the flow rate of the culture solution reaches 1,500 mL/h/m², but an increment in the flowing-out amount slows down at 6,000 mL/h/m² or more. 1,500 mL/h/m² or more is preferable. The flow rate of the culture solution is a value measured at any location on the surface of the carrier 2.

If the flow rate is too large, there are problems in that microorganisms such as algae are unlikely to be fixed to the carrier 2, the growth rate decreases, the nutrient solution phase becomes thick, CO₂ exchange becomes difficult, and stress is applied to microorganisms such as algae due to physical stimulation.

If the carrier 2 is made of a twisted yarn or untwisted yarn pile fabric of 0.5 m² or more, the flow rate of the culture solution flowing on the surface of the carrier 2 is a rate of higher than 1,200 mL/h/m², and is preferably 5,400 mL/h/m² or more and more preferably 9,000 mL/h/m² or more. The upper limit of the flow rate is preferably 30,000 mL/h/m² or less, more preferably 27,000 mL/h/m² or less, and still more preferably 24,000 mL/h/m² or less. The flow rate of the culture solution can be measured as well as above.

The culture solution is not particularly limited as far as it is a culture medium diluted solution in which microorganisms are cultured by a generally used method and the concentration of microorganisms can be increased. As the culture medium, a general inorganic culture medium, for example, a CHU culture medium, a JM culture medium, or an MDM culture medium, can be used. In addition, as the culture medium, diluted solutions of various culture mediums such as a Gamborg B5 culture medium, a BG11 culture medium, and an HSM culture medium are preferable. The inorganic culture medium contains Ca(NO₃)₂·4H₂O, KNO₃, or NH₄Cl as a nitrogen source, and KH₂PO₄, MgSO₄·7H₂O, FeSO₄·7H₂O or the like as other main nutrient components. Antibiotics that do not influence the growth of microorganisms and the like may be added to the culture medium. The pH of the culture medium is preferably 4 to 10. If possible, wastewater and the like discharged from various industrial facilities may be used.

The sheet component 4 is a translucent sheet-like member formed of a synthetic resin such as vinyl, polyethylene, polyester, or the like. When microorganisms that can grow without photosynthesis are cultured using the culture system 1, the material of the sheet component 4 does not need to be transparent.

The sheet component 4 has a rectangular parallelepiped bag shape that is disposed to wrap the carrier 2, and includes a rectangular front surface part 4a and rear surface part 4b that are disposed in parallel at certain intervals from the front surface and the rear surface of the carrier 2, side surface parts 4c and 4c that connect both side edges of the front surface part 4a and the rear surface part 4b, and a bottom surface part 4d that connects lower ends of the front surface part 4a and the rear surface part 4b.

The front surface part 4a and the rear surface part 4b are folded back in an inverted U shape at the upper end and are continuous, and the folded part is hung on the culture solution supply unit 3, and additionally is attached to the culture solution supply unit 3 with a fastener (not shown).

Between the folded part of the front surface part 4a and the rear surface part 4b and the side surface part 4c, an insertion hole 15 through which the culture solution supply unit 3 passes and an insertion hole 16 through which the fixing member 10 of the hanger H passes are formed. In the bottom surface part 4d, a lead-out hole 17 through which a culture solution containing microorganisms flowing down from the carrier 2 is led to the effluent tank 5 is formed. In the present embodiment, the lead-out hole 17 has an elongated and substantially rectangular shape (slit shape), but the lead-out hole 17 may be a round hole or a hole with another shape, and the number of holes may be one or plural. A lead-out nozzle (not shown) that guides the culture solution to the effluent tank 5 may be attached to the lead-out hole 17.

The sheet component 4 may have a form in which it covers the top and sides of the carrier 2 in a tubular shape including parts other than the lower part of the leg member 11 of the member that supports the carrier 2 such as the hanger H, and opens downward only, the leg member 11 is caused to protrude, and the culture solution can be led out. In order to keep the carrier 2 warm, maintain carbon dioxide gas in the atmosphere to which the carrier 2 is exposed, and prevent scattering of a harvested component scraped from the carrier 2, the inside of the sheet component 4 is preferably sealed or almost sealed. The sheet component 4 may be fixed to the case 8 to be described below with a fastener or the like.

The sheet component 4 is preferably disposed in the vicinity of the surface of the carrier 2. "The vicinity" in the present invention means that the component is installed with a separation distance between the sheet component 4 and the surface of the carrier 2 that is in a range of 1 to 10 cm.

When the sheet component 4 is installed in this range, it is possible to effectively keep the surface of the carrier 2 warm. If the distance between the sheet component 4 and the surface of the carrier 2 is less than 1 cm, the sheet component 4 may be brought into close contact with the surface of the carrier 2 due to slight shaking or vibration, and there is a risk of a culture solution or gas not being sufficiently supplied to the contact part and the growth of microorganisms being delayed. If the distance between the sheet component 4 and the surface of the carrier 2 exceeds 10 cm, a sufficient warming effect may not be obtained. The distance between the sheet component 4 and the surface of the carrier 2 is more preferably in a range of 1 to 5 cm.

When a plurality of carriers 2 are provided, the sheet component 4 may be installed to cover the carriers 2, and one large sheet component 4 may be installed to cover the plurality of carriers 2 together.

The effluent tank 5 is a tank for storing a culture solution containing microorganisms flowing out from the carrier 2 and has a box shape with a certain depth of which the upper end is opened so that the culture solution flowing down from the carrier 2 is received. Due to gravity, the culture solution containing microorganisms flowing out from the carrier 2 is separated into a precipitate containing a high concentration of microorganisms and a culture solution which is a supernatant containing almost no microorganisms in the effluent tank 5.

The harvest container 6 is a container in which the precipitate containing a high concentration of microorganisms separated in the effluent tank 5 is collected from the bottom of the effluent tank 5 and stored.

Through the circulation flow path 7, the culture solution (supernatant) separated in the effluent tank 5 is collected and supplied to the carrier 2 again. A pump P is provided at the circulation flow path 7, and thereby the collected culture solution can be pumped up to the above the carrier 2. The pumped up culture solution is continuously supplied again from above the carrier 2. The culture solution supplied to the carrier 2 is a supernatant separated in the effluent tank 5 but it may contain microorganisms. A strainer may be provided in front of the pump P and at least some of microorganisms contained in the supernatant may be collected by filtering with the strainer.

The case 8 of this embodiment has a box shape and covers the entire carrier 2, culture solution supply unit 3, sheet component 4, effluent tank 5, and circulation flow path 7. As long as the case 8 covers at least the carrier 2 and the sheet component 4, it is not essential that it cover other members. When the sheet component 4 and the carrier 2 are covered with the case 8, the heat retention ability is further improved and the surface temperature of the carrier 2 can be easily kept constant.

The material of the case 8 is not particularly limited, and examples thereof include transparent materials such as glass, acrylic, polystyrene, and vinyl chloride. When microorganisms that can grow without photosynthesis are cultured using the culture system 1, the material of the case 8 does not need to be transparent. When a plurality of sets of the sheet component 4 and the carrier 2 are installed in the case 8, the effluent tank 5, the harvest container 6, and the circulation flow path 7 may not be provided for each individual set but can be shared.

The light emission unit 9 is a member that emits light to the carrier 2 from the front surface, and includes, for example, as a light source, a plate-like fluorescent lamp, an organic EL or LED, or the like, and appropriately emits light with a wavelength and light intensity suitable for growth. The wavelength of light that the light emission unit 9 emits may be in a range of 380 to 780 nm. The light emission unit 9 may emit only red light suitable for photosynthesis to microorganisms that can grow with only red light. Microorganisms such as *Chlorellae* can grow well with only red light. Emission of light from the light emission unit 9 may not be continuous emission but may be intermittent emission of light of 100 to 10,000 Hz. The culture system 1 can be installed outdoors and use sunlight. It is also effective to introduce sunlight while the system is installed indoors and combine it with artificial light.

When the carrier 2 has a cylindrical shape or a rectangular tube shape, a light emission unit 9 with a rod shape or the like may be additionally disposed in the carrier 2 so that light is also emitted to the inner circumferential surface of the carrier 2.

While Fig. 1 shows a state in which the light emission unit 9 is installed outside the case 8, the light emission unit 9 may be installed in the case 8.

In the example shown in Fig. 1, one light emission unit 9 is provided in the culture system 1, and emits light to one entire surface of the carrier 2 almost uniformly. A plurality of light emission units 9 may be provided according to the number of carriers 2. When a plurality of carriers 2 and light emission units 9 are provided, the carriers 2 and the light emission units 9 can be alternately installed in parallel. In this case, if the light emission unit 9 is a double-sided light emission unit, it can emit light to the carriers 2 on both sides.

In the case 8, an air mixture containing about 1 to 40% CO₂ is filled, and additionally, in order to appropriately replenish CO₂, CO₂ or air containing CO₂ can be preferably fed. In this embodiment, an introducing nozzle 4e through which CO₂ is introduced from an external carbon dioxide gas source is installed at the lower part of the sheet component 4, and an exhaust nozzle 4f through which an inner gas is discharged to the outside is installed at the upper part of the sheet component 4. When the carrier 2 is kept in an air mixture containing about 1 to 10% CO₂, many microorganisms can perform photosynthesis favorably. Even if air is ventilated inside the sheet component 4, the growth of microorganisms is possible although the speed is slow.

In the culture system 1 shown in Fig. 1, a heat retention property can be improved using the sheet component 4 and the case 8. However, in the microorganism culture system of the present invention, as far as an atmospheric temperature and atmospheric gas are maintained in appropriate ranges for the growth, microorganisms can be cultured without using the sheet component 4 and the case 8.

In the present invention, microorganisms that are a culture subject are preferably photosynthetic microorganisms, and in this case, the culture system 1 preferably includes the light emission unit 9. When microorganisms that can grow without photosynthesis are cultured, or when photosynthetic microorganisms are cultured outdoors, the light emission unit 9 may be omitted.

Next, the usage method and operation of the culture system 1 will be described.

In order to start use of the culture system 1, absorbent cotton or the like to which microorganisms are adhered is placed on the carrier 2, and its end is hung on or suspended and fastened by the hanger H or the like. As a method of adhering microorganisms, microorganisms may be directly dropped or applied to the carrier 2. Around the carrier 2, the sheet component 4 is installed at a position in a range of 1 cm or more and 10 cm or less from the surface of the carrier 2. CO₂ or air containing about 1 to 40% CO₂ is introduced from the introducing nozzle 4e, and is caused to flow from the bottom to the top, and excess gas is discharged from the discharge nozzle 4f.

Then, red light with a wavelength of 380 to 780 nm is emitted while a culture solution is continuously supplied so that it flows down on the surface of the carrier 2 from the culture solution supply unit 3 at a rate of 5 mL/h/m² or more. In this light emission, at the beginning of seeding of microorganisms, the light intensity is weak at about 50 µmol m⁻²s⁻¹ and is increased to about 400 µmol m⁻²s⁻¹ as they grow. In addition, since photosynthetic organisms have a characteristic of dividing at night, it is preferable to set a light-out time at the initial stage of growth. In this case, a liquid temperature and an atmospheric temperature on the surface of the carrier 2 are preferably set to 33 to 37 °C

When a certain time has elapsed, the culture solution spreads throughout the carrier 2, the culture solution is additionally supplied from the culture solution supply unit 3, and the culture solution flows down from the lower end of the carrier 2 to the effluent tank 5. When microorganisms are gradually cultured on the carrier 2, microorganisms adhered to the carrier 2 or microorganisms cultured in the carrier 2 gradually flow out from the carrier 2 due to the flow of the culture solution and flow down to the effluent tank 5 together with the culture solution.

Microorganisms flowing down from the carrier 2 are precipitated in the culture solution in the effluent tank 5, and taken into the harvest container 6 installed below the effluent tank 5. On the other hand, a supernatant of the culture solution containing some of the microorganisms stored in the effluent tank 5 is pumped up by the pump P, sent again to the culture solution supply unit 3 through the circulation flow path 7, and supplied to the carrier 2 repeatedly.

The amount of a new culture solution supplied from the culture solution reservoir tank (not shown) is adjusted according to the amount of the culture solution containing microorganisms supplied again to the culture solution supply unit 3. In addition, required nutrients are appropriately supplied to the culture solution supply unit 3 from a nutrient supply tank (not shown), and are discharged to the carrier 2 together with the culture solution.

As described above, some of the microorganisms naturally flow down from the carrier 2. However, in this embodiment, according to division and growth of microorganisms, the surface layer of the microorganism layer fixed on the carrier 2 may be scraped off. Therefore, photosynthesis is activated in microorganisms in the lower layer part and division and growth start.

When the above operations are repeated, microorganisms are continuously cultured and the cultured microorganisms are harvested.

According to the culture system 1 of the present embodiment, since a sheet or a pile fabric having a water capacity of 0.2 g/cm² or more per unit area is used as the carrier 2, compared to a case using a conventional culture system with the same size, the amount of microorganisms produced increases, microorganisms can be harvested regularly in a short period, and microorganism production efficiency can be improved.

Here, the present invention is not limited to the above embodiment. For example, microorganisms may be collected from the culture solution stored in the effluent tank 5 through any of filtration, centrifugation, and natural sedimentation. When substances that are discharged from microorganisms to the outside of cells are harvested, other methods such as adsorption and concentration are applied.

While a vertically long carrier is provided in the embodiment, a horizontally long carrier may be used. In addition, without departing from the spirit and scope of the present invention, some or all of the various configurations above may be appropriately combined.

### EXAMPLES

While the present invention will be described below in more detail with reference to examples, the present invention is not limited to these examples.

### [Water retention test]

In this specification, "water capacity" of the carrier was measured by the following method.
[1] A sample for measuring a water capacity with a size of 3 cm×26 cm was prepared and a dry weight was measured.
[2] The sample was put into a container containing a sufficient amount of tap water at room temperature (for example, 23 °C) and left for 3 minutes, and sufficient water was incorporated into the sample.
[3] One end of the sample in the longitudinal direction was pinched using tweezers, then the sample was stretched and removed from the container in the vertical direction and left for 5 seconds while lifted from the surface of water until water dripped.
[4] The weight of the sample containing water was measured. Even if water dripped at that time, the weight including dripping water was measured.
[5] The amount of water contained per 1 cm² of the sample was calculated by subtracting the dry weight of the sample from the weight measured in [4].

Measurement was performed five times for each sample, and the average value thereof was used as the "water capacity (g/cm²)."

### [Example 1]

*Chlorellae* (*Chlorella kessleri* 11h) were cultured using the culture system 1 shown in Fig. 1. As the carrier 2, a carrier in which a pile fabric (water capacity: 0.395 g/cm²) woven with untwisted yarns with a width of 50 cm×a length of 120 cm was folded in half and suspended on the fixing member 10 of the hanger H was used.

As the sheet component 4 covering the carrier 2, a commercially available vinyl chloride sheet with a width of 80 cm×a height of 90 cm×a thickness of 1.2 mm was used, and covered the entire carrier 2 and a part of the culture solution supply unit 3. The pump P (product name "1046" commercially available from EHEIM) was provided downstream from the circulation flow path 7. A commercially available glass case (the thickness of the glass was 3 mm) was used as the case 8. A red LED (commercially available from Effect) was used as the light emission unit 9.

Using this culture system 1, air containing 10 volume% CO₂ was introduced at a rate of about 1.0 L/min into the case 8 from the bottom to the top, and bubbled in the culture solution in the effluent tank 5. As the culture solution, a solution in which KNO₃ was added to a plant tissue culture medium Gamborg B5 that was diluted 50-fold so that the concentration was 150 mg/L was used. *Chlorellae* were cultured at 33 to 37 °Cwhile the culture solution was supplied to the carrier 2 at a rate of 1,000 mL/h and red light with an intensity of 50 µmol m⁻²s⁻¹ was emitted to the carrier 2. When the culture started, *Chlorellae* with a dry weight of 15 g were adhered to absorbent cotton placed on the carrier 2, and then the culture started.

Two hours after the culture started, the concentration of the culture medium was adjusted so that the Gamborg B5 culture medium was diluted 10-fold. From the next day, the culture medium in the effluent tank 5 was replaced once a day with a culture solution obtained by adding, per liter of the culture medium, 750 mg of KNO₃, 5 ml of a nutrition reinforcing agent (composition for returning to a 10-fold diluted Gamborg B5 culture medium, and including NaH₂PO₄: 17 g/1, MgSO₄: 15 g/l, and (NH₄)₂SO₄: 13 g/l, and not containing glucose), and 50 µl of NH₄Cl to the solution in which the Gamborg B5 culture medium was diluted 10-fold. The light intensity was set to 100 µmol m⁻²s⁻¹ from the day after the culture started. The surface temperature of the carrier 2 was constant at 33 to 35 °Cduring the culture.

The culture solution containing *Chlorellae* flowing out from the carrier 2 was collected in the effluent tank 5. In order to prevent *Chlorellae* in the effluent tank 5 from growing, the effluent tank 5 was covered with a black cloth. In collection of *Chlorellae*, the surface of the carrier 2 was scraped off 1 to 3 times a day from the 3^{rd} day after the culture started, and the culture solution in the effluent tank 5 was centrifuged. The collected *Chlorellae* were re-suspended in the culture solution, and the dry weight was calculated from the turbidity at 730 nm (turbidity of 0.35 at 730 nm=1 g DW (dry weight)/L) measured using a spectrophotometer (DU700 commercially available from Beckman Coulter, Inc.). In addition, the dry weight was determined from *Chlorellae* dried at 80 °C for 2 hours or longer and the calculated value was corrected. As a result of the culture, *Chlorellae* with a dry weight of 169.56 g/m² were harvested on the 5^{th} day after the culture started (calculated per 1 m² of the carrier 2).

### [Example 2]

*Chlorellae* were cultured and the dry weight was calculated in the same manner as in Example 1 except that a pile fabric woven with twisted yarns with a width of 50 cm×a length of 120 cm (water capacity: 0.267 g/cm²) was used as the carrier 2. As a result of the culture, *Chlorellae* with a dry weight of 157.07 g/m² were harvested on the 5^{th} day after the culture started.

### [Comparative Example 1]

*Chlorellae* were cultured and the dry weight was calculated in the same manner as in Example 1 except that a non-woven fabric with three pieces of gauze with a width of 45 cm×a length of 160 cm (water capacity: 0.185 g/cm²) was used as the carrier 2. As a result of the culture, *Chlorellae* with a dry weight of 112.21 g/m² were harvested on the 5^{th} day after the culture started.

In Examples 1 and 2 in which a pile fabric having a water capacity per unit area of 0.2 g/cm² or more was used as the carrier, the amount of *Chlorellae* produced was significantly larger than that in Comparative Example 1 in which a non-woven fabric with three pieces of gauze was used as the carrier.

### [Second embodiment]

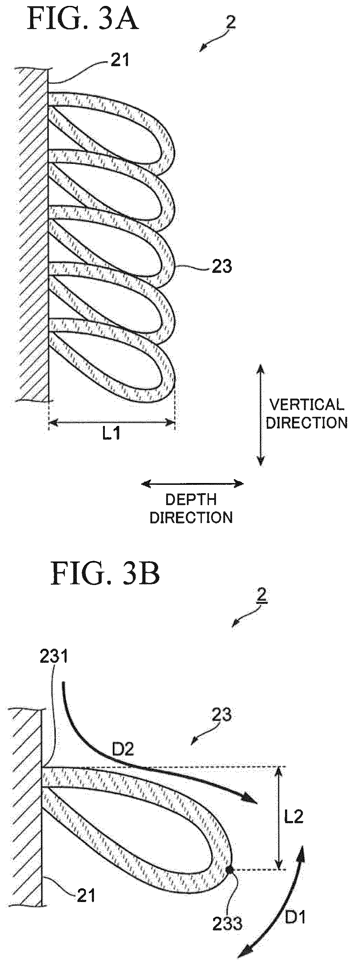

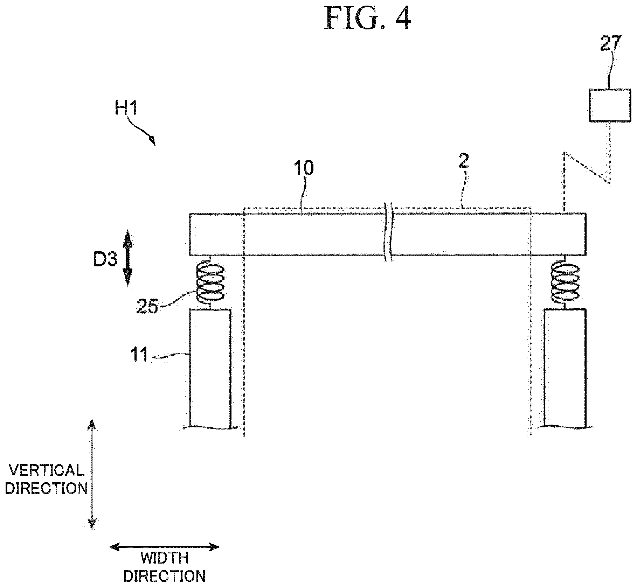

Fig. 3A is an enlarged cross-sectional view of a carrier 2 according to a second embodiment, and Fig. 3B is an illustrative diagram of the protrusion part 23. The carrier 2 is suspended on a fixing member 10 of a hanger H1 (refer to Fig. 4).

Fig. 4 is a schematic configuration diagram of the hanger H1 according to the second embodiment.

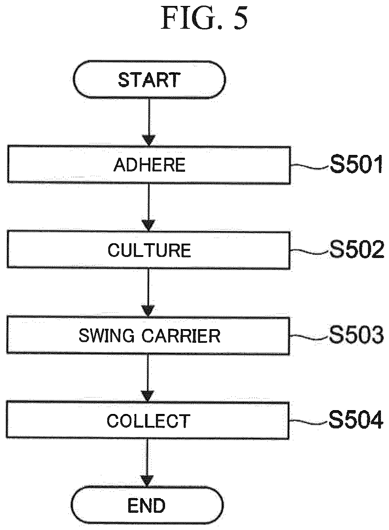

Fig. 5 is a flowchart illustrating a microorganism culture collection method according to the second embodiment.

Next, the second embodiment will be described with reference to Fig. 3A, Fig. 3B, Fig. 4, and Fig. 5. In the following description, the same components as those described in the above first embodiment will be denoted with the same reference numerals and detailed descriptions thereof will be omitted.

### [Carrier 2]

First, the carrier 2 according to the second embodiment will be described with reference to Fig. 3A and Fig. 3B. The carrier 2 can be used in the first embodiment or other embodiments to be described below.

The carrier 2 is formed of a pile fabric. The carrier 2 shown in Fig. 3A includes a base 21 which is a flat plate part formed of fibers, and a fibrous member that protrudes from the surface of the base 21, that is, a plurality of protrusion parts 23 formed of a raised part. The illustrated protrusion part 23 has an annular part (loop part). The protrusion part 23 has a root 231 fixed to the base 21 and a tip 233 which is a part of the protrusion part 23 that is farthest from the base 21. For example, the protrusion part 23 has a fiber diameter of 1 mm, and has an amount of protrusion from the base 21 (the length in the depth direction, refer to distance L1) of about 20 mm. In addition, as described in the above first embodiment, a water capacity per unit area of the carrier 2 is 0.2 g/cm² or more.

As shown in Fig. 3B, as the protrusion part 23 is elastically deformed, the tip 233 of the protrusion part 23 is movable with respect to the root 231. That is, the tip 233 can swing around the root 231 (refer to arrow D1), and is movable in a direction (in the drawing, up and down and left to right directions) along the base 21 and also movable in an advancing and retreating direction (in the drawing, the depth direction) with respect to the base 21.

As shown in Fig. 3B, when the carrier 2 is suspended, that is, the base 21 is disposed in the vertical direction, the tip 233 can be positioned below the root 231 (refer to distance L2). In this manner, if a culture solution is supplied to the carrier 2 when the tip 233 is disposed below the root 231, the culture solution flows from the root 231 toward the tip 233 (refer to arrow D2). In this manner, since the culture solution flows toward the tip 233, the culture solution and microorganisms are prevented from remaining on the side of the root 231. As a result, deterioration of the culture solution and microorganism that can occur due to the remaining culture solution and microorganisms and the like can be prevented.

In the carrier 2 according to the present embodiment, when the protrusion part 23 is provided on the base 21, the surface area of the carrier 2 increases. Therefore, in the culture solution present inside the carrier 2 or on the surface of the carrier 2, an area of a region in contact with a gas phase increases and a light-receiving area of the carrier 2 also increases. Accordingly, the growth of microorganisms in the carrier 2 can be promoted.

In order to increase the surface area of the carrier 2, instead of a configuration in which the protrusion part 23, that is, a protrusion, is provided in the carrier 2 as in the present embodiment, for example, a configuration in which a concave section is provided like a porous sponge can be assumed. However, in this aspect, pores in the carrier formed in a sponge shape are filled with the culture solution, and the culture solution and microorganisms may remain inside the pores. If the culture solutions and microorganisms inside the pores are not discharged, the above-described deterioration of the culture solution and microorganism and the like may occur. In addition, in the configuration in which a concave section is provided like a porous sponge, it is not possible to adhere a sufficient amount of microorganisms. On the other hand, in the present embodiment, when the protrusion part 23 as a protrusion is formed as described above, the culture solution and microorganisms flow on the surface of the protrusion part 23. As a result, deterioration of the culture solution and microorganisms and the like are prevented.

The shape of the protrusion part 23 is not particularly limited as long as the tip 233 is movable with respect to the root 231 (the base 21). For example, unlike the illustrated example, the protrusion part 23 may not be formed in an annular shape. For example, the protrusion part 23 may be a substantially cylindrical, substantially conical, or substantially prismatic member of which one end is supported on the base 21 and the other end is swingable. For example, the carrier 2 may be formed of a shearing fabric. The shearing fabric is a fabric obtained by cutting off the tip of the loop of thread on one side of the pile fabric, and a large number of linear fibers protrude from the ground weave instead of a loop-like thread. The pile length of the shearing fabric is not particularly limited, and can be 3 mm to 30 mm.

### [Hanger H1]

Next, a hanger H1 will be described with reference to Fig. 4. The hanger H1 can be used as a hanger in the first embodiment or other embodiments to be described below.

As shown in Fig. 4, the hanger H1 includes the rod-like fixing member 10 that is horizontally disposed, the leg members 11 that support both ends of the fixing member 10, a buffer mechanism 25 that is provided between the fixing member 10 and the leg member 11, and a frame drive source 27 that is connected to the fixing member 10 and vibrates the fixing member 10. The hanger H1 and the carrier 2 are examples of a carrier unit. The frame drive source 27 is an example of a swinging mechanism.

The buffer mechanism 25 prevents vibration of the fixing member 10 from being transmitted to the leg members 11. The illustrated buffer mechanism 25 is formed of a spring member. As the buffer mechanism 25, other elastic components such as a rubber, a hydraulic mechanism such as a damper, and the like may be used.

The frame drive source 27 swings the fixing member 10. The illustrated frame drive source 27 is composed of a vibration motor that generates a vibration by rotating a rotation shaft in which an eccentric vibrator is provided. An air vibrator, an acoustic vibrator or the like may be used as the frame drive source 27. The period and intensity of vibration are set to the extent at which microorganisms are appropriately shaken off from the carrier 2.

### [Culture collection method]

Next, a microorganism culture collection method (culture subject collection method) in the second embodiment will be described with reference to Fig. 3A, Fig. 3B, Fig. 4, and Fig. 5. In the following description, the carrier 2 is suspended on the fixing member 10 of the hanger H1.

First, as described in the above first embodiment, microorganisms are adhered to the carrier 2 (step 501). Light is emitted while supplying the culture solution to the carrier 2 from the culture solution supply unit 3 (an example of the supply unit, refer to Fig. 1), and microorganisms are cultured in the carrier 2 (step 502). Next, as the frame drive source 27 is driven and the fixing member 10 is swung, the carrier 2 swings (step 503). Although the details will be described later, as the carrier 2 swings, the culture solution and microorganisms adhered to the carrier 2 are separated from the carrier 2. The culture solution and microorganisms separated from the carrier 2 are collected using the effluent tank 5 (for an example of the reservoir unit, refer to Fig. 1) (step 504).

When the frame drive source 27 is driven, a vibration is transmitted to the fixing member 10. In the illustrated example, the fixing member 10 vibrates in the vertical direction (refer to arrow D3). As the fixing member 10 vibrates, the carrier 2 swings. When the fixing member 10 vibrates in the vertical direction, an effect of shaking off microorganisms is thought to be improved as will be described below.

When the carrier 2 swings vertically, the protrusion part 23 provided on the base 21 swings in the vertical direction. That is, the tip 233 of the protrusion part 23 swings vertically, and a relative position of the tip 233 with respect to the root 231 (the base 21) varies. When the protrusion part 23 swings, the culture solution and microorganisms are swung and separated from the tip 233 of the protrusion part 23, and microorganisms adhered to the carrier 2 are separated from the carrier 2. Accordingly, it is possible to prevent an excessive amount of microorganisms from remaining on the carrier 2 and nutrients and light can be supplied to new microorganisms always.

In the illustrated example, the buffer mechanism 25 is provided between the fixing member 10 and the leg member 11. Therefore, even if the fixing member 10 receives a vibration from the buffer mechanism 25, it is possible to prevent other members such as the leg member 11 from swinging. When other members such as the leg member 11 are allowed to swing, the buffer mechanism 25 may not be provided.

When the frame drive source 27 is driven to swing the carrier 2 (step 503), the supply of the culture solution may be continued or stopped. In addition, when the frame drive source 27 is driven, while the inside of the sheet component 4 (refer to Fig. 1) is filled with the culture solution and the carrier 2 is immersed in the culture solution, the carrier 2 is swung and microorganisms may be shaken off. In addition, a direction in which the frame drive source 27 is driven to move the fixing member 10 is not limited to the vertical direction. For example, the direction may be the width direction or the depth direction, or a combination of some or all of the vertical direction, the width direction, or the depth direction.

### [Third embodiment]

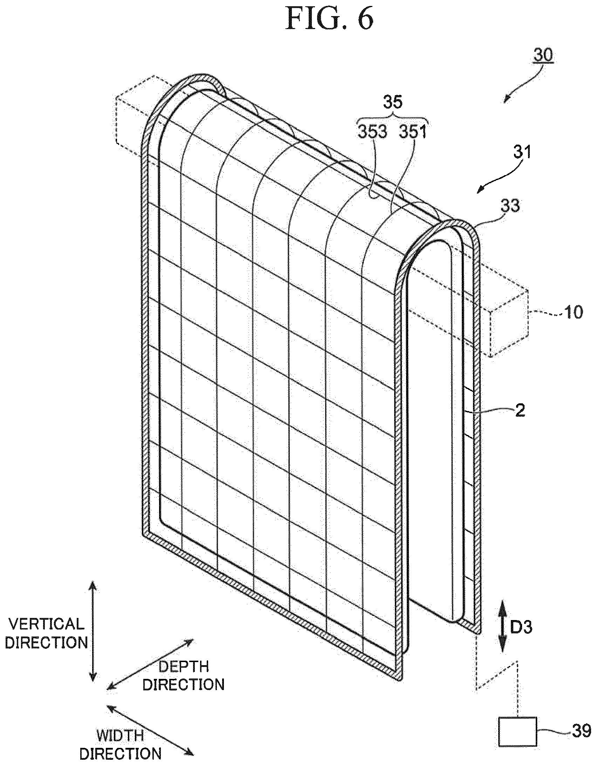

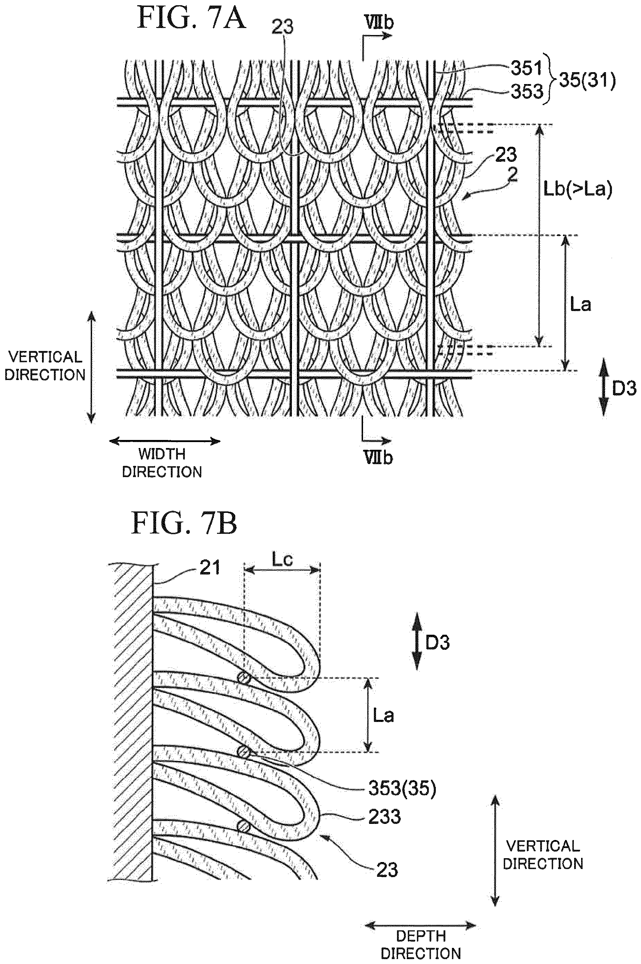

Fig. 6 is a schematic configuration diagram of a net vibration mechanism 30 according to a third embodiment. Fig. 7A is an enlarged view of a net component 31 and the carrier 2, and Fig. 7B is a cross-sectional view along the line VIIb-VIIb in Fig. 7A.

The third embodiment will be described with reference to Fig. 6, Fig. 7A, and Fig. 7B. In the following description, the same components as those described in the first and second embodiments will be denoted with the same reference numerals and detailed descriptions thereof will be omitted.

### [Net vibration mechanism 30]

As shown in Fig. 6, in the third embodiment, the net vibration mechanism 30 for swinging the surface of the carrier 2 is provided. The net vibration mechanism 30 includes the net component 31 that is provided to cover the entire surface of the carrier 2 and a net component drive source 39 that is connected to the net component 31 and vibrates the net component 31. In Fig. 6, for clarification, the net component 31 is separated from the carrier 2, but when the net component 31 is mounted on the carrier 2, the net component 31 comes in contact with the outer circumferential surface of the carrier 2.

The net component 31 as an example of a contact member includes a frame part 33 and a lattice part 35 that is supported by the frame part 33.

The frame part 33 is formed of a metallic rod-like member made of, for example, stainless steel (SUS), in a rectangular shape, and has a shape and size along the outer circumference of the carrier 2 hung on the fixing member 10. That is, the frame part 33 is formed in a rectangular shape slightly larger than the carrier 2, and is bent in the longitudinal direction at the center and formed in substantially a U shape in a side view. The material and size of the frame part 33 are not particularly limited as long as it has a rigidity at which the lattice part 35 can be supported.

The lattice part 35 has a vertical line 351 and a horizontal line 353 provided in directions that are orthogonal to (intersect) each other. In the illustrated example, the vertical line 351 extends in the vertical direction and a plurality of vertical lines are arranged in parallel at predetermined intervals. On the other hand, the horizontal line 353 extends in the width direction, and a plurality of horizontal lines are arranged in parallel at predetermined intervals. The vertical line 351 and the horizontal line 353 constituting the lattice part 35 are composed of a linear member (for example, synthetic fiber thread) that can transmit light emitted from the light emission unit 9 (refer to Fig. 2). For example, the lattice part 35 may be made of a transparent resin or glass. When the lattice part 35 has a light transmission property, light from the light emission unit 9 passes through the vertical line 351 and the horizontal line 353 constituting the lattice part 35 and reaches the carrier 2.

The net component drive source 39 as an example of a moving unit is composed of, for example, a rack and pinion, and a motor that drives a rack gear. In the illustrated example, when the motor of the net component drive source 39 is driven, the net component 31 swings in the vertical direction (refer to arrow D3). Accordingly, the net component 31 vibrates the protrusion part 23.

### [Operation of net component 31]

Next, the operation of the net component 31 will be described with reference to Fig. 6, Fig. 7A, and Fig. 7B. The horizontal lines 353 of the net component 31 are provided at predetermined intervals (refer to the distance La in Fig. 7A) as described above. In addition, the net component 31 receives a driving force of the net component drive source 39 and swings in the vertical direction (reciprocating movement, refer to arrow D3). Then, the horizontal line 353 of the net component 31 swings in a direction (vertical direction) crossing the direction (width direction) in which the horizontal line 353 extends. In this case, in the vertical direction, the length of the region through which one horizontal line 353 passes, that is, a swing range (the length Lb in Fig. 7A) for one horizontal line 353, is larger than the interval (distance La) between the horizontal lines 353. That is, the horizontal line 353 reciprocates in a range larger than one grid of the lattice forming the net component 31. For example, in this configuration, the horizontal line 353 moves twice the interval (refer to distance La) between the horizontal lines 353, that is, two grids of the lattice. In this manner, when each horizontal line 353 reciprocates in the vertical direction in a range lager than one grid of the lattice, the horizontal line 353 can rub against the entire surface of the carrier 2.

As shown in Fig. 7B, when the net component 31 is mounted on the carrier 2, the horizontal line 353 of the net component 31 is disposed closer to the base 21 (refer to distance Lc) than the tip 233 of the protrusion part 23. When the culture solution is supplied to the carrier 2 and the protrusion part 23 is wet with the culture solution, the horizontal line 353 of the net component 31 is disposed closer to the base 21 than the tip 233 of the protrusion part 23. In other words, the lattice part 35 is interposed between the protrusion parts 23. In this manner, when the lattice part 35 starts to move from the position at which it is disposed between the protrusion parts 23, the lattice part 35 can reliably press the protrusion part 23. As a result, the protrusion part 23 can reliably swing vertically and a larger amount of microorganisms can be separated from the carrier 2. As shown in Fig. 6, when the net component 31 covers the outer circumferential surface of the carrier 2 in this configuration, the lattice part 35 can rub against the entire outer circumferential surface of the carrier 2.

As in the microorganism culture collection method described in Fig. 5, also in the third embodiment, microorganisms are cultured and collected. However, unlike the second embodiment, when the carrier is swung (step 503 in Fig. 5), the net vibration mechanism 30 swings the net component 31. As the carrier 2 swings, microorganisms are separated from the carrier 2.

When the net component 31 is mounted on the carrier 2, that is, the lattice part 35 is interposed between the protrusion parts 23, microorganisms may be cultured in the carrier 2, or after microorganisms are cultured in the carrier 2 without mounting the net component 31, the net component 31 may be mounted to cover the carrier 2.

While the lattice part 35 formed of a material having a light transmission property has been described in the above embodiment, the present invention is not limited thereto. For example, a thin rod-like member (wire) formed of stainless steel (SUS) may be used as the lattice part 35. In addition, the lattice part 35 having the vertical line 351 and the horizontal line 353 that are provided in directions orthogonal to each other has been described. However, only one of the vertical line 351 and the horizontal line 353 may be provided or a linear member that extends in a direction other than the vertical line 351 and the horizontal line 353 may be provided. When only one of the vertical line 351 and the horizontal line 353 is provided, swinging may occur in a direction orthogonal to the longitudinal direction.

Other driving mechanisms such as a solenoid and a vibration motor may be used as the net component drive source 39. When the net component drive source 39 is driven, for example, while the inside of the sheet component 4 is filled with the culture solution, and the carrier 2 is immersed in the culture solution, the net component 31 may be swung. A direction in which the net component drive source 39 is driven to move the net component 31 is not limited to the vertical direction. For example, the direction may be the width direction or the depth direction, or a combination of some or all of the vertical direction, the width direction, or the depth direction.

### [Fourth embodiment]

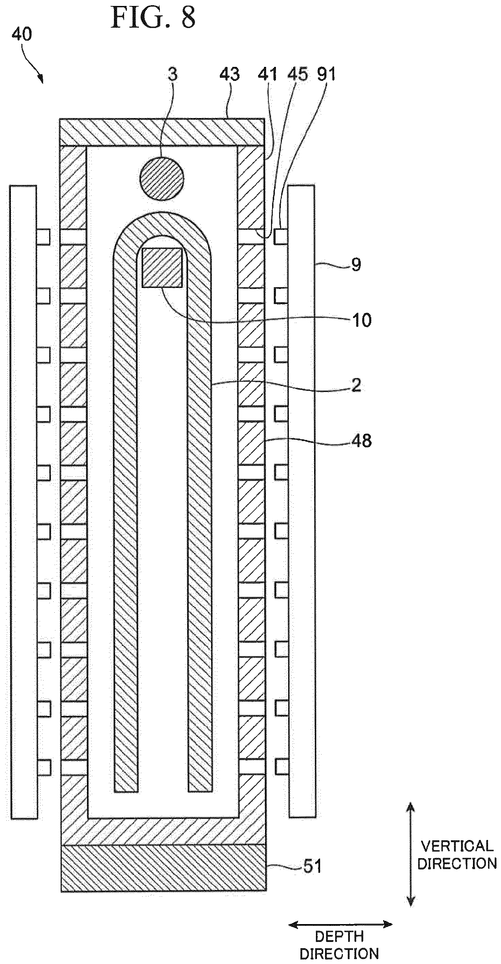

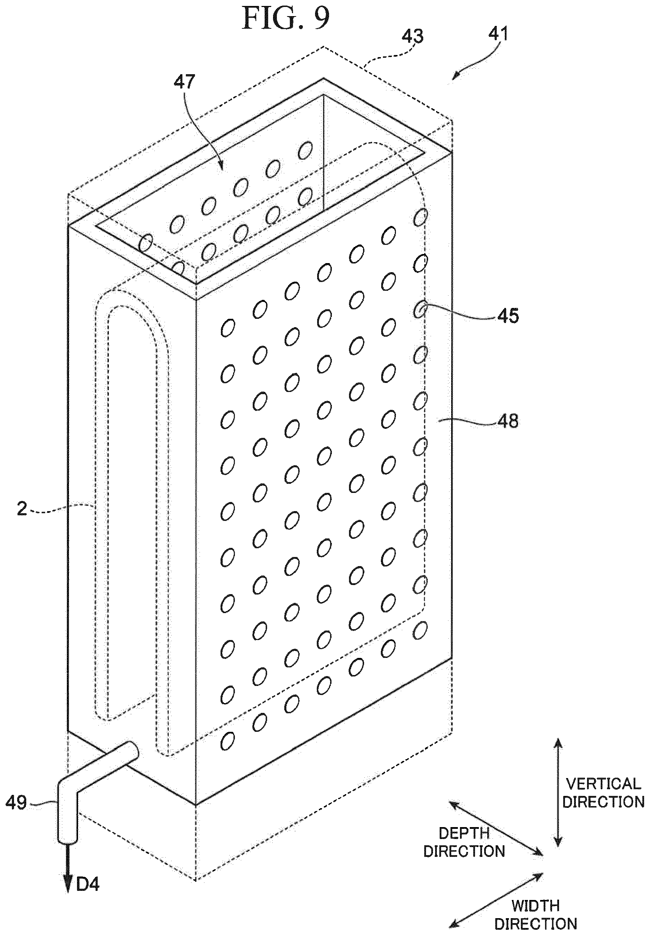

Fig. 8 is a schematic configuration diagram of an ultrasonic vibration mechanism 40 according to a fourth embodiment. Fig. 9 is a perspective view of a vibration tank 41 in the fourth embodiment.

Next, the fourth embodiment will be described with reference to Fig. 8 and Fig. 9. In the following description, the same components as those described in the first to third embodiments will be denoted with the same reference numerals and detailed descriptions thereof will be omitted.

### [Ultrasonic vibration mechanism 40]

As shown in Fig. 8, in the fourth embodiment, the ultrasonic vibration mechanism 40 that swings the surface of the carrier 2 is provided. The ultrasonic vibration mechanism 40 includes the vibration tank 41 in which the carrier 2 is stored therein, a covering member 43 that covers the upper side of the vibration tank 41, and an ultrasonic vibrator 51 that vibrates the vibration tank 41.

In the fourth embodiment, the vibration tank 41 as an example of a storage unit is provided instead of the sheet component 4 (refer to Fig. 1) in the first embodiment. As shown in Fig. 9, the vibration tank 41 is a substantially rectangular parallelepiped hollow member and an opening 47 (refer to Fig. 9) is formed on the upper side. The vibration tank 41 is formed of, for example, stainless steel. The vibration tank 41 has a side surface 48 on which a plurality of transparent windows 45 are formed. At the bottom of the vibration tank 41, a discharge mechanism 49 that discharges (refer to arrow D4) the culture solution and microorganisms from the inside of the vibration tank 41 is formed. Each transparent window 45 is provided at a position facing each LED 91 of the light emission unit 9. Each transparent window 45 is covered with a resin that transmits light emitted from the LED 91. The transparent window 45 transmits light emitted from the LED 91 and allows light to reach the carrier 2 disposed inside the vibration tank 41.

The covering member 43 is provided above the vibration tank 41 and covers the opening 47. Accordingly, the internal space of the vibration tank 41 is sealed and the temperature and atmosphere inside the vibration tank 41 are easily controlled. The covering member 43 is made of, for example, a metal such as stainless steel or a resin. The ultrasonic vibrator 51 as an example of an ultrasonic vibration unit is provided below the vibration tank 41. The frequency of ultrasonic waves generated from the ultrasonic vibrator 51 is not particularly limited, and is, for example, 10 kHz to 100 kHz. In the illustrated example, the ultrasonic vibrator 51 generates ultrasonic waves with a frequency of 20 kHz.

### [Operation of ultrasonic vibration mechanism 40]

Next, the operation of the ultrasonic vibration mechanism 40 will be described. In the following description, the carrier 2 is stored in the vibration tank 41 and the opening 47 of the vibration tank 41 is then covered with the covering member 43. In the fourth embodiment, as in the microorganism culture collection method described above in Fig. 5, microorganisms are cultured and collected.

In the fourth embodiment, unlike the second embodiment, when the carrier 2 is swung (refer to step 503 in Fig. 5), the inside of the vibration tank 41 is filled with the culture solution, and the carrier 2 is immersed in the culture solution. Then, when the carrier 2 is immersed in the culture solution and the ultrasonic vibrator 51 is driven, ultrasonic waves that are transmitted through the culture solution allow the carrier 2 to swing. Accordingly, microorganisms adhered to the carrier 2 are separated from the carrier 2. The culture solution containing microorganisms is discharged from the inside of the vibration tank 41 through the discharge mechanism 49 and collected (refer to step 504 in Fig. 5) in the effluent tank 5 (refer to Fig. 1).

The ultrasonic vibrator 51 may be any vibrator that can swing the carrier 2 via the culture solution, and may be provided, for example, on the side of or above the vibration tank 41. A so-called throw-in ultrasonic generating device may be used as the ultrasonic vibrator 51. That is, the ultrasonic vibrator 51 may be provided inside the vibration tank 41 and a flow type ultrasonic generating device may be used as the ultrasonic vibrator 51. In addition, the present invention is not limited to a configuration using ultrasonic waves, and the carrier 2 may be swung using other waves such as shock waves.

In the above embodiment, the entire carrier 2 is stored in the vibration tank 41, but the present invention is not limited thereto. For example, a mode in which a part of the carrier 2 protrudes outside the vibration tank 41 may be used. In this configuration, the carrier 2 may be formed in a belt shape that is long in one direction and a part of the belt-like carrier 2 may be disposed inside the vibration tank 41. When the carrier 2 formed in a belt shape is used, a mechanism that transmits the carrier 2 is provided, and thus a part that is disposed inside the vibration tank 41 can be changed, and a part in which microorganisms are cultured and a part in which microorganisms are collected can be separated. In this manner, in the configuration in which a part in which microorganisms are cultured and a part in which microorganisms are collected are separated, it is not necessary to emit light to the carrier 2 inside the vibration tank 41 in which microorganisms are collected. Thus, in such a configuration, the transparent window 45 may not be provided in the vibration tank 41.

### [Culture subject]

The culture subject that the culture system 1 as an example of a culturing device cultures as described above includes not only photosynthetic microorganisms having no or poor mobility such as *Chlorellae*, *Synechocystis*, and *Spirulina* but also planktonic *Euglena*, *Chlamydomonas*, and *Pleurochrysis* which move in water with flagella. Microorganisms to be cultured in the culture system 1 are very diverse. Examples of main microorganism groups to be cultured in the culture system 1 include the following species A, species B, species C, and the like.

First, examples of species A include eubacteria and archaebacterial which are prokaryotes.

Examples of eubacteria include non-oxygen-producing photosynthetic bacteria and oxygen-producing photosynthetic cyanobacteria, facultative anaerobic fermenting bacteria and non-fermenting bacteria using organic substances, and additionally autotrophic bacteria, actinomycetes and corynebacterium, and spore-bearing bacteria. Examples of photosynthetic bacteria include rhodobacter, rhodospirillum, chlorobium, and chloroflexus. Examples of cyanobacteria include synechococcus, synechocystis, spirulina, arthrospira, nostoc, anabaena, oscillatoria, lyngbya, nostoc commune, and aphanothece sacrum.

Examples of facultative anaerobic fermenting bacteria include *E. coli*, and lactic acid bacteria. Examples of non-fermenting bacteria include *pseudomonas.* Examples of autotrophic bacteria include hydrogen bacteria. Examples of actinomycetes include *Streptomyces* and examples of spore-bearing bacteria include *Bacillus subtilis.* Examples of archaebacterial include thermophilic bacteria and extremely halophilic bacteria. Examples of thermophilic bacteria include *Thermococcus*, and examples of extremely halophilic bacteria include *halobacterium.* Other examples include glutamic acid-producing bacteria, lysine-producing bacteria, and cellulose-producing bacteria.

Next, examples of species B include microalgae which are eukaryotic photosynthetic microorganisms.

Examples of microalgae include green algae, *Trebouxia* algae, red algae, *Diatoms*, haptophytes, eustigmatophytes, *Euglena*, and zooxanthellae.

Examples of green algae include *Chlorellae*, *Scenedesmus*, *Chlamydomonas*, *Botryococcus*, *Haematococcus*, *Nannochloris*, and *Pseudochoricystis*, and examples of *Trebouxia* algae include *Parachlorella* and *Coccomyxa.* Examples of red algae include *Cyanidioschyzon merolae*, *Cyanidium*, *Galdieria*, and *Porphyridium*, and examples of *Diatoms* include *Nitzschia*, *Phaeodactylum*, *Chaetoceros*, *Thalassiosira*, *Skeletonema*, and *Fistulifera.* Examples of haptophytes include *Pleurochrysis*, *Gephyrocapsa*, *Emiliania*, *Isochrysidales*, and *Pavlova.* Examples of eustigmatophytes include *Nannochloropsis*, and examples of *Euglena* include *Euglena.* In addition, examples of zooxanthellae as coral symbiotic algae include *Cymbiodinium.*

Next, examples of species C include fungi which are non-photosynthetic eukaryotes. Examples of fungi include yeasts and *Aspergillus.* In addition, the mycelial culture of basidiomycetes is the subject of culture.

Although they are not microorganisms, *Ulva* and *Enteromorpha* which are green algae among multicellular seaweeds, *Porphyra tenera*, *Porphyra*, *Porphyra yezoensis*, and *Collema* which are red algae, and other edible seaweeds can also be the culture subject. In addition, hepatophyta which are chlorophytes can also be the culture subject. In addition, Lichenobionta which are symbionts can also be the culture subject. Microalgae include cyanobacteria.

### [Other]

As described above, the present invention provides a culture system in which microorganisms grow on the surface of and inside a carrier and microorganisms can be efficiently produced. The inventors conducted extensive studies in order to address the above problems and as a result, found that, if a component having a water capacity of 0.2 g/cm² or more per unit area or pile fabric is used as the carrier, microorganisms can be efficiently produced, and completed the present invention. Specifically, the present invention includes the following aspects.
[1] A microorganism culture system (1) includes a carrier (2) which is disposed in a gas phase and to which microorganisms adhere, a culture solution supply unit (3) that supplies a culture solution from above the carrier (2), and an effluent tank (5) in which the culture solution containing microorganisms flowing out from the carrier (2) is stored, and a water capacity per unit area of the carrier (2) is 0.2 g/cm² or more.
[2] A microorganism culture system (1) includes a carrier (2) which is disposed in a gas phase and to which microorganisms adhere, a culture solution supply unit (3) that supplies a culture solution from above the carrier (2), and an effluent tank (5) in which the culture solution containing microorganisms flowing out from the carrier (2) is stored, and a water capacity per unit area of the carrier is 0.25 g/cm² or more.
[3] A microorganism culture system (1) includes a carrier (2) which is disposed in a gas phase and to which microorganisms adhere, a culture solution supply unit (3) that supplies a culture solution from above the carrier (2), and an effluent tank (5) in which the culture solution containing microorganisms flowing out from the carrier (2) is stored, and a water capacity per unit area of the carrier is 0.3 g/cm² or more.
[4] The microorganism culture system according to any one of (1) to (3), wherein the carrier (2) is a pile fabric.
[5] The microorganism culture system according to any one of [1] to [3], wherein the carrier (2) is a pile fabric woven with untwisted yarns.
[6] A microorganism culture system (1) includes a carrier (2) which is disposed in a gas phase and to which microorganisms adhere, a culture solution supply unit (3) that supplies a culture solution from above the carrier (2), and an effluent tank (5) in which the culture solution containing microorganisms flowing out from the carrier (2) is stored, and the carrier (2) is a pile fabric.
[7] The microorganism culture system according to [6], wherein the pile fabric is a pile fabric woven with untwisted yarns.
[8] The microorganism culture system according to any one of [1] to [7], wherein the microorganisms are microalgae.

In the microorganism culture system of the present invention, when a component having a water capacity of 0.2 g/cm² or more per unit area or pile fabric is used as the carrier, compared to production using a conventional microorganism culture system with the same size, the amount of microorganisms produced can significantly increase and microorganisms can be harvested regularly in a very short period, and thus an effect of further improving the production efficiency compared with a conventional method is obtained.

While various embodiments and modified examples have been described above, these embodiments and modified examples may be combined to each other in the configuration. In addition, the present disclosure is not limited to the above embodiments, and can be implemented in various forms without departing from the spirit and scope of the present disclosure.

### [Industrial Applicability]

According to the technology disclosed in this specification, microorganisms can be efficiently produced on the surface or the inside of the carrier and collected so that the technology can be industrially used.

### [Reference Signs List]

1 Culture system
2 Carrier
3 Culture solution supply unit
6 Harvest container
7 Circulation flow path
8 Case
21 Base
23 Protrusion part
27 Frame drive source
30 Net vibration mechanism
31 Net component
40 Ultrasonic vibration mechanism

## Claims

1. A culturing device, comprising:
a carrier unit having a carrier to which a culture subject adheres;
a supply unit that supplies a culture solution to the carrier; and
a reservoir unit in which the culture solution flowing out from the carrier is stored,
wherein the carrier includes
a base, and
a plurality of protrusions which are formed on the surface of the base and of which a relative position of each tip with respect to the base is able to be changed, and
wherein the carrier unit includes a swinging mechanism that swings the carrier and changes the relative positions of the protrusions.

2. The culturing device according to Claim 1, wherein the base is provided in a vertical direction and the tips of the protrusions are positioned below roots of the protrusions.

3. The culturing device according to Claim 2, wherein the supply unit is configured so that the culture solution supplied from the supply unit to the carrier flows from the roots of the protrusions toward the tips.

4. The culturing device according to Claim 1, wherein the protrusions are fibrous members that extend from the base.

5. The culturing device according to Claim 4, wherein the fibrous members have annular parts.

6. The culturing device according to any one of Claims 1 to 5,
wherein the swinging mechanism includes
a storage unit in which the carrier is stored, and
an ultrasonic vibration unit that applies an ultrasonic vibration to the carrier stored in the storage unit.

7. The culturing device according to any one of Claims 1 to 5,
wherein the swinging mechanism includes
a contact member that is provided in contact with the surface of the carrier, and a moving unit that moves the contact member along the surface.

8. A culturing device, comprising:
a carrier to which a culture subject adheres;
a supply unit that supplies a culture solution to the carrier; and
a reservoir unit in which the culture solution flowing out from the carrier is stored,
wherein a water capacity per unit area of the carrier is 0.2 g/cm² or more.

9. A carrier to be used for a culturing device including a carrier to which a culture subject adheres, a supply unit that supplies a culture solution to the carrier, and a reservoir unit in which the culture solution flowing out from the carrier is stored, the carrier comprising:
a base having a sheet form; and
a plurality of protrusions which are formed on the surface of the base and of which a relative position of each tip with respect to the base is able to be changed,
wherein the tips of the protrusions are able to be positioned below roots of the protrusions.

10. A culture subject collection method, comprising the steps of:
adhering a culture subject to a carrier including a base and a plurality of protrusions which are formed on the surface of the base and of which a relative position of each tip with respect to the base is able to be changed;
culturing the culture subject in the carrier;
changing the relative positions of the protrusions by swinging the carrier and separating the culture subject from the carrier; and
collecting a fluid containing the culture subject separated from the carrier.
